Europäisches Patentamt

European Patent Office

Office européen des brevets

(11) Veröffentlichungsnummer: **0 436 766 A1**

## (12) EUROPÄISCHE PATENTANMELDUNG

(21) Anmeldenummer: 90100648.6

(22) Anmeldetag: 12.01.90

(51) Int. Cl.⁵: **A61B 6/00**

(43) Veröffentlichungstag der Anmeldung:
17.07.91 Patentblatt 91/29

(84) Benannte Vertragsstaaten:
**DE FR GB**

(71) Anmelder: **Siemens Aktiengesellschaft**
**Wittelsbacherplatz 2**
**W-8000 München 2(DE)**

(72) Erfinder: **Romándi, Dénes, Dipl.-Ing.**
**Steigerwaldallee 7A**
**W-8520 Erlangen(DE)**

(54) **Medizinische Diagnostikanlage.**

(57) Bei einer medizinischen Diagnostikanlage mit mindestens einem fernsteuerbaren medizintechnischen Gerät (1) und einer losgelöst davon handhabbaren Bedienvorrichtung (7) erfolgt eine drahtlose Übertragung von Steuerbefehlen. Diese Übertragung erfolgt gleichzeitig durch zwei unterschiedliche Medien, vorzugsweise durch Infrarot- und Ultraschallstrahlung, so daß eine störungsfreie Informationsübertragung sichergestellt ist.

EP 0 436 766 A1

## MEDIZINISCHE DIAGNOSTIKANLAGE

Es ist bekannt, in der Medizintechnik Informationen mit Hilfe von Kabeln oder Lichtwellenleitern zu übertragen. Auf diese Weise werden z.B. Röntgengeräte ferngesteuert. Auch die Übertragung der Signale des Strahlendetektors eines Röntgenbelichtungsautomaten zu der zugeordneten Schaltung, die Strahlungsauslösung und die Monitorbedienung kann dadurch erfolgen.

Die feste Verdrahtung ist oft störend und auch störempfindlich. Deshalb können die Komponenten einer medizintechnischen Diagnostikanlage auch drahtlos ferngesteuert werden.

Die drahtlose Fernsteuerung auf Infrarot- oder Ultraschallbasis ist z.B. aus der Unterhaltungselektronik bekannt. Bisher wurde dem Einsatz in der Medizintechnik mit Vorsicht begegnet, weil beide Übertragungsmedien gestört werden können, z.B. Infrarotstrahlung durch Strahlungsanteile im Spektrum von Leuchtstoffröhren und Ultraschallstrahlung durch spektrale Anteile in Raumgeräuschen.

Der Erfindung liegt die Aufgabe zugrunde, eine medizintechnische Diagnostikanlage mit mindestens einem fernsteuerbaren medizintechnischen Gerät so auszubilden, daß Störungen bei der Informationsübertragung mit Sicherheit vermieden sind.

Diese Aufgabe ist erfindungsgemäß dadurch gelöst, daß die Übertragung der Steuerbefehle gleichzeitig durch zwei unterschiedliche Medien, vorzugsweise durch Infrarot- und Ultraschallstrahlung erfolgt. Eine solche Diagnostikanlage ermöglicht es, die Ausführung eines Steuerbefehles nur dann zuzulassen, wenn die von einem Infrarotempfänger und von einem Ultraschallempfänger gleichzeitig empfangene Information identisch ist. Treten Störungen auf dem Infrarotweg oder auf dem Ultraschallwegauf, so wird die Ausführung des Steuerbefehles verhindert. Die Wahrscheinlichkeit, daß beide Kanäle gleichzeitig im gleichen Sinne gestört werden, ist vernachlässigbar gering.

Durch die Erfindung kann eine einfache, mobile und doch sehr störsichere Verbindung zwischen einer Bedienvorrichtung und einer drahtlos ferngesteuerten Komponente realisiert werden.

Die Erfindung ist nachfolgend anhand eines in der Zeichnung dargestellten Ausführungsbeispieles näher erläutert. Es zeigen:

Fig. 1 eine Röntgendiagnostikanlage nach der Erfindung, und

Fig. 2 eine Einzelheit der Röntgendiagnostikanlage nach Fig. 1.

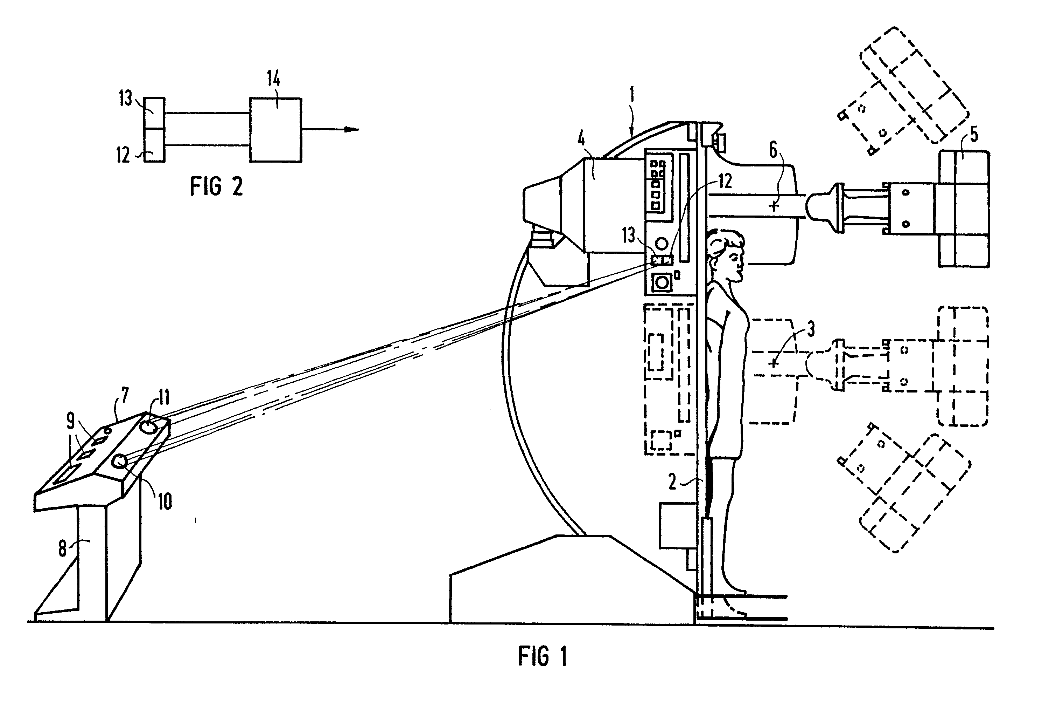

In der Fig. 1 ist ein drahtlos fernsteuerbares Röntgendiagnostikgerät 1 dargestellt, das eine in Richtung ihrer Längsrichtung verstellbare Lagerstatt 2, die auch um eine horizontale Achse 3 kippbar ist, aufweist. Für die Bilderzeugung ist ein Bildaufnahmesystem mit einem Röntgenbildverstärker 4 und einem Röntgenstrahler 5 vorhanden, welches verschiedene Stellungen einnehmen kann. Es ist in der Längsrichtung der Patientenlagerstatt 2 verschiebbar sowie um eine horizontale Achse 6 kippbar. In der Fig. 1 sind verschiedene mögliche Stellungen der Patientenlagerstatt 2 und des Bildaufnahmesystems 4, 5 gestrichelt dargestellt.

Die Übertragung von Steuerbefehlen zur Einstellung des Röntgengerätes 1 erfolgt in drahtloser Weise mit Hilfe einer Bedienvorrichtung 7, die auf einer Ablage 8 ruht und von dort abnehmbar sein kann. Die Bedienvorrichtung 7 weist Bedienelemente 9 für die Steuerbefehle sowie einen Infrarotsender 10 und einen Ultraschallsender 11 auf. Die Steuerbefehle werden gleichzeitig durch Infrarot- und Ultraschallstrahlung zu einem Infrarotempfänger 12 und einem Ultraschallempfänger 13 am Röntgengerät 1 übertragen.

Die Fig. 2 zeigt, daß der Infrarotempfänger 12 und der Ultraschallempfänger 13 an einer Erkennungsschaltung 14 angeschlossen sind. Die Erkennungsschaltung 14 läßt die Ausführung eines Steuerbefehles nur zu, wenn die vom Infrarotempfänger 12 und vom Ultraschallempfänger 13 gleichzeitig empfangene Information identisch ist. Auf diese Weise ist eine äußerst störsichere Informationsübertragung von der Bedienvorrichtung 7 zum Röntgengerät 1 sichergestellt.

Die Erfindung ist in Verbindung mit der Fig. 1 anhand eines fernsteuerbaren Röntgendiagnostikgerätes zur Lagerung und Untersuchung eines Patienten beschrieben. Erfindungsgemäß können auch andere medizintechnische Geräte, z.B. Röntgengeneratoren, Röntgenfernsehanlagen oder dergleichen in der beschriebenen Weise mit zwei unterschiedlichen Medien ferngesteuert werden. So können z.B. die Aufnahmedaten für den Röntgengenerator mit einer losgelöst davon handhabbaren Bedienvorrichtung für die drahtlose Übertragung von Steuerbefehlen eingestellt werden.

**Patentansprüche**

1. Medizinische Diagnostikanlage mit mindestens einem fernsteuerbaren medizintechnischen Gerät (1) und einer losgelöst davon handhabbaren Bedienvorrichtung (7) für die drahtlose Übertragung von Steuerbefehlen, die gleichzeitig durch zwei unterschiedliche Medien erfolgt.

2. Medizinische Diagnostikanlage nach Anspruch 1, bei der die Medien Infrarot- und Ultraschallstrahlung sind.

3. Medizinische Diagnostikanlage nach Anspruch 1 oder 2 mit einer Erkennungsschaltung (14), die die Ausführung eines Steuerbefehles nur zuläßt, wenn die von einem Infrarotempfänger (12) und von einem Ultraschallempfänger (13) gleichzeitig empfangene Information identisch ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | PATENT ABSTRACTS OF JAPAN Band 11, Nr. 170 (P-581), 2. Juni 1987; & JP - A - 62002183 (SHARP CORPORATION) 08.01.1987 * Zusammenfassung; Figur * | 1 | A 61 B 6/00 |
| A | idem <br> --- | 2,3 | |
| Y | DE-A-3 036 217 (SIEMENS AG) * Seite 11, Zeile 29 - Seite 12, Zeile 13; Figuren 1-3 * | 1 | |
| A | --- | 2 | |
| A | ELEKTOR Band 9, Nr. 11, November 1983, Seiten 1158,1159, Canterbury, GB; "ultrasonic/infrared barrier" * Seite 1158; Figuren 1,2 * <br> ----- | 1-3 | |

RECHERCHIERTE SACHGEBIETE (Int. Cl.5)

A 61 B 6/00
H 03 K 17/00

Der vorliegende Recherchenbericht wurde für alle Patentansprüche erstellt

| Recherchenort | Abschlußdatum der Recherche | Prüfer |
|---|---|---|
| BERLIN | 22-08-1990 | WEIHS J.A. |